Europäisches Patentamt

European Patent Office

Office européen des brevets

(11) Publication number: **0 451 687 A2**

# EUROPEAN PATENT APPLICATION

(21) Application number: **91105284.3**

(22) Date of filing: **03.04.91**

(51) Int. Cl.⁵: **G01N 33/569,** //G01N33/538, G01N33/543

(30) Priority: **12.04.90 US 508817**

(43) Date of publication of application:
**16.10.91 Bulletin 91/42**

(84) Designated Contracting States:
**DE FR GB IT**

(71) Applicant: **MILES INC.**
**1127 Myrtle Street**
**Elkhart Indiana 46514(US)**

(72) Inventor: **Messenger, Koleen K.**
**17453 Green Oaks Ct.**
**Granger, IN 46530(US)**
Inventor: **Miller, Carol A.**
**51101 Winding Water Lane N.**
**Elkhart, IN 46514(US)**
Inventor: **Sharma, Harmesh K.**
**17420 Parker**
**South Bend, IN 46635(US)**
Inventor: **Thompson, Stephan G.**
**1210 E. Wayne**
**South Bend, IN 56615(US)**

(74) Representative: **Dänner, Klaus, Dr. et al**
**c/o Bayer AG Konzernverwaltung RP**
**Patentabteilung**
**W-5090 Leverkusen 1 Bayerwerk(DE)**

(54) **Chlamydia half-sandwich immunoassay.**

(57) A method for detecting the presence of Chlamydia trachomatis in a test sample, such as a clinical specimen containing infected human cells, in which the test sample is treated to expose multivalent Chlamydia antigens from C. trachomatis present in the sample and a labeled antibody reagent is added to form a mixture comprising immune complexes of the labeled antibody and released antigens. The mixture is passed through a glass fiber depth filter that is impervious to the resulting immune complexes and the label that is retained by the filter is detected. The half-sandwich method is sensitive and rapid, and requires a minimum number of reagents and assay steps.

EP 0 451 687 A2

BACKGROUND OF THE INVENTION

The present invention relates to the detection of Chlamydia trachomatis, particularly in biological specimens. More particularly, the invention relates to immunoassay detection of C. trachomatis in a human urogenital sample.

Chlamydia trachomatis is a Gram-negative bacterium which is the causative agent in a number of human disease syndromes. Chlamydial infections of the urogenital tract are the fastest growing of the sexually transmitted diseases. Its detection is clinically important since infections are often unaccompanied by symptoms. A general review of the laboratory detection and diagnosis of human Chlamydial infections is provided by Barnes, Clin. Microbiol. Rev. 2:119-136 (1989).

A variety of methods have been developed over the years for the detection of C. trachomatis, including culture methods, immunoassays, and DNA probe assays. Immunoassay methods based on the specific detection of antigens of the Chlamydia organism are most commonly used in the clinical diagnosis of Chlamydial infection. Such immunoassay methods include direct fluorescent assays, in which Chlamydia antigens exposed on cells fixed to a microscope slide are detected by binding of fluorescently-labeled antibodies, and by enzyme immunoassays (EIA) in which Chlamydia antigens released from a clinical specimen are detected by binding of enzyme-labeled antibodies.

The presently available commercial EIA tests for Chlamydia fall into two general categories, the sandwich EIA and the method described in U.S. Pat. No. 4,497,889. In the sandwich EIA method, released Chlamydia antigen is reacted with an enzyme-labeled antibody and a solid-phase antibody resulting in the formation of an insoluble complex of labeled antibody bound to Chlamydia antigen bound, in turn, to the solid-phase antibody. After removal of unbound labeled-antibody, the presence of enzyme on the solid phase, due to the presence of Chlamydia antigen in the sample, is determined by adding a chromogenic substrate and observing the appearance of color. Performance of the sandwich EIA method involves a number of sequential, time consuming steps. Examples of sandwich EIAs for detecting C. trachomatis are described in U.S. Pat. Nos. 4,727,019 and 4,830,960.

A different EIA principle is described in the aforementioned U.S. Pat. No. 4,497,899. In this method, Chlamydia antigen is released from a cellular test sample into a test mixture and adsorbed nonspecifically to what is described as a bare solid support, a support that does not comprise Chlamydia antibody as in the solid-phase antibody reagent used in the sandwich EIA method. After separating the solid support from the test mixture, enzyme-labeled antibody is added which will bind to Chlamydia antigen adsorbed to the support. Enzyme present on the solid support is then detected with a chromogenic substrate as in the sandwich EIA method. While operating on a different assay principle, this EIA method nonetheless requires performance of a number of sequential, time consuming steps as in sandwich EIA.

It is an object of the present invention to provide an immunoassay method which requires a minimum number of reagents and assay steps. Such a method would be particularly advantageous for performance of Chlamydia assays by relatively untrained individuals such as in a physician's office or small clinical laboratory, or by patients themselves.

SUMMARY OF THE INVENTION

It has been unexpectedly found that the simple and rapid immunoassay detection of Chlamydia trachomatis in a test sample can be accomplished by (a) treating the sample to form a liquid test mixture containing exposed multivalent C. trachomatis antigens, (b) contacting the exposed multivalent Chlamydia antigens with a labeled, e.g., enzyme-labeled, antibody reagent that binds to said antigens to form immune complexes, (c) passing the resulting liquid mixture through a glass fiber depth filter that is substantially impervious to such immune complexes but which does not retain significant amounts of unbound labeled antibody reagent, (d) passing a wash solution through the glass fiber filter effective to remove substantially all unbound labeled antibody reagent present thereon after completion of step(c),and (e) detecting the presence of such immune complexes retained by the filter by observing or measuring the label, e.g., in the case of an enzyme label, by applying a chromogenic substrate to the filter and observing or measuring any resulting color. The method is particularly useful for detecting Chlamydia in a clinical specimen, e.g., a urogenital specimen, that comprises potentially infected human cells. Exposure of antigens is usually accomplished by treating the specimen to rupture human cells sufficiently to release C. trachomatis therefrom and to disrupt the cytoplasmic membrane of any such released C. trachomatis to expose multivalent antigens. The multivalent antigens of interest are typically found in outer membrane proteins and lipopolysaccharides (LPS).

The present method is referred to herein as a "half-sandwich " immunoassay since the present method involves only a single assay step and a single binding reagent (the labeled antibody) in comparison to both the prior art sandwich and adsorption EIA methods wherein multiple steps are performed in order to bind Chlamydia antigens to two different reagents or test means (i.e., the labeled and solid-phase antibodies in the sandwich method and the labeled antibody and bare support in the adsorption method). As a result, the present method is characterized by a number of significant advantages. The method requires fewer reagents and assay steps, and particularly fewer steps requiring timing by the user, which is of critical importance in the performance of such immunoassay methods by relatively untrained individuals such as in a physician's office or small clinical laboratory, or by patients themselves. The total time for the assay is equivalent, or in some cases shorter, than for prior art methods. Further, the present assay has been demonstrated to be more sensitive than prior art methods and is certainly easier to manufacture, there being no need to prepare solid-phase antibody reagents or special bare supports, or devices carrying same.

BRIEF DESCRIPTION OF THE DRAWINGS

Fig. 1 is an isometric projection of a test device useful in performing the Chlamydia assay of the present invention.

Fig. 2 is an exploded diametric projection of the test device shown in Fig. 1.

Fig. 3 is a cross-sectional view of the device shown in Fig. 1.

Figs. 4 and 5 are top plan views of the Fig. 1 device, with Fig. 5 showing the device with its funneling element removed.

Figs. 6, 6a, and 6b show different test responses that are produced using the device of Fig. 1 in the present assay method.

DESCRIPTION OF THE PREFERRED EMBODIMENTS

It is critical to the present invention that the immune complexes formed by binding between the anti-Chlamydia antibody reagent and the Chlamydia antigens released from the test specimen be substantially retained by the glass fiber depth filter while unbound labeled antibody reagent substantially passes through the filter. As will be brought out more fully below, this critical separation of bound from unbound labeled antibody reagent has been found to be dependent on a number of controllable factors, including the nature of the Chlamydia antigens detected, the size and configuration of the labeled antibody reagent, the porosity of the glass fiber filter, and the presence of interfering substances in the test sample or generated in the sample processing step which can be retained by the filter and bind the labeled antibody nonspecifically. Considering, among other factors, the nature of the test sample involved and the criticality of the separation of bound from unbound labeled reagent, it was not evident prior to the successful performance of the method of the present invention that diagnostically significant results could be obtained in this manner for the detection of Chlamydia trachomatis.

The first step in the present method, sometimes referred to herein as the sample processing step, involves the treatment of the test sample to expose detectable amounts of multivalent Chlamydia antigens. C. trachomatis is an obligate intracellular bacterium exhibiting a unique biphasic growth cycle. The two representative forms of the bacterium are the elementary body (EB) and the reticulate body (RB). The metabolically inactive, but infectious, EB form invades columnar epithelial cells and undergoes transformation to the metabolically active RB form. Both forms of the bacterium comprise a number of antigenic components, including a major outer membrane protein which is morphologically associated with the porin structure of the outer membrane, lipopolysaccharide (LPS) antigens, and others (Barnes, supra). Any such antigens, or complexes or fragments thereof that can be generated from the test sample, can serve as detectable antigens in the present method provided that such are multivalent, that is, capable of being bound by multiple antibody reagents in order to form filter-retainable immune complexes.

In the case of clinical specimens that comprise human cells, the sample processing step serves two principle purposes: (1) rupture of human eucaryotic cell membranes to release intracellular Chlamydia EB and RB forms, and (2) disruption, with or without lysis, of the cytoplasmic membrane of released EBs and RBs to make the necessary antigenic sites or epitopes available for binding by the antibody reagent. Sample treatment will thus vary according to the antigenic sites or epitopes of interest. The prior art methods typically use relatively harsh conditions for sample processing, e.g., 1 M sodium hydroxide or 0.5 M dithiothreitol (DTT), in order to fully solubilize membrane components. However, whereas such prior art sample processing agents can be washed away prior to the addition of the immunoassay reagents, the present method requires that residual sample processing agents in the processed test mixture be compatible with the labeled antibody reagent since the labeled reagent is added directly thereto. Accordingly, the sample processing conditions must be selected to enable release of the detectable Chlamydia antigens without denaturing or otherwise interfering or inhibiting either the binding of labeled antibody to such released antigens or the detectable activity of the label, e.g., an enzyme.

With these considerations in mind, the ordinary skilled worker can empirically select suitable and optimum sample processing agents and conditions. Such agents will comprise detergents, including ionic and nonionic, high and low salt concentrations, high and low pH, common organic solvents, such as dimethylsulfoxide (DMSO) and dimethylformamide (DMF), buffer ions, such as phosphate, carbonate, and borate, and the like.

As an example, where the antigen to be exposed is an outer membrane protein, a useful sample processing liquid can be buffered to a pH between about 8 and about 9, preferably with a TRIS buffer (e.g., at about 0.1 M). Also useful for exposing such a protein antigen are any one or more of the following: reducing agents such as acetylcysteine (e.g., at concentrations below 200 mM); enzymes such as chondroitinase, neuraminidase, and trypsin; halide salts (e.g., at concentrations between 0.1 and 1 M); oxidizing agents such as hydrogen peroxide; and detergents such as Sarkosyl (e.g., at concentrations below 0.5%), sodium dodecylsulfate (SDS) (e.g., at concentrations below 0.1%), and Triton X-100 (e.g., at concentrations below 0.5%). Where both a reducing agent and an oxidizing agent are used, normally the former will be added to the specimen first and after a reaction period, the latter added.

As a further example, where the antigen to be exposed is an LPS, a useful sample processing liquid can be buffered to between about 6.5 and about 9, preferably with a phosphate buffer (e.g., 0.1 M). Also useful for exposing such an LPS antigen are any one or more of the following: reducing agents such as DTT (e.g., at concentrations below 50 mM) and acetylcysteine (e.g., at concentrations below 100 mM); halide salts (e.g., at concentrations below 0.5 M); and detergents such as deoxycholate (e.g., at concentrations below 0.035%).

A clinical test sample can be obtained from a patient in a variety of forms. While specimens can be collected from other sites, of most significance is the collection of urogenital specimens. The most common form of sample for testing with the present method will be cervical or urethral swabs. Conveniently, swabs can be placed in a sample processing tube or other suitable vessel containing the desired sample processing agents as described above. Prior to being removed from the tube, the swab will normally be compressed to expel as much sample material as possible into the sample processing mixture. For example, the swab can be squeezed against the inside wall of the sample processing tube, or the tube can comprise structures such as ridges or constrictions, against or through which the swab must be pushed and/or pulled in order to fully insert it into and/or remove it from the tube.

The labeled antibody reagent can be added with the sample processing agent in one step, or can be added afterwards to the liquid test mixture formed by action of the sample processing agent on the test sample, optionally with an intermediate or subsequent filtration step. Since the test specimen, particularly clinical specimens such as urogenital specimens, will normally contain a variety of cellular debris as well as mucus, filtration of some or all of such material prior to the ultimate passage of the liquid test sample through the glass fiber separation filter is often desirable or necessary. Such a pre-filtration step can assure that the separation filter does not become plugged and the flow of the test mixture therethrough significantly reduced or prevented. Moreover, in some cases, sample debris, if not pre-filtered, can collect on the separation filter and interfere with the separation of bound and unbound labeled antibody, increase nonspecific binding of the labeled antibody to the filter, or cause non-uniform generation of signal from the label.

A pre-filter will be selected to remove unwanted or unnecessary sample materials from the test sample or mixture without substantially removing Chlamydia antigens to be detected, or labeled antibody reagent when the pre-filtration is performed after addition thereof. Typically, pre-filtration is accomplished by pushing a piston-type pre-filter down the specimen processing tube, or other reaction vessel being used, and thus through the liquid test sample or mixture, leaving the filtered liquid above the filter and available for the next step. Nominal pore size will generally be in the range of from about 10 to about 150 microns. A number of suitable filters are commercially available, for example, ultra high molecular weight polyethylene depth filters (available from Porex Technologies Corp., Fairburn, GA, USA), #903 filters from Schleicher & Schuell (Keene, NH, USA), hydrophilic HDC filters from Pall Biosupport (Glen Cove, NY, USA), and spunbound polyester filters Reemay available from Reemay, Inc. (Old Hickory, TN, USA). The pre-filtration step can be performed either before or after addition of the labeled antibody reagent to the liquid test sample, the former being preferred.

The labeled antibody reagent binds directly to Chlamydia antigens in the test mixture in order to form labeled immune complexes which are separable from the labeled reagent itself upon passage of the mixture through the glass fiber depth filter. It is this interaction which is principally responsible for the simplicity and ease of use of the present method. The labeled reagent is prepared with these functions in mind. Preferably, the reagent will comprise multiple antigen-combining sites to increase the macromolecular immune network complexes that can be formed with the multivalent Chlamydia antigens in the processed sample, although monovalent antibody fragments such as Fab' have been shown to be operable. A variety of methods will be evident for increasing the valency of the labeled antibody reagent, for example, polymerization or aggregation of antibody or antibody fragments, coupling of multiple antibodies or antibody fragments to a carrier molecule, e.g., a synthetic or natural polymer backbone, or to the label or multiples of the label where the label is suitable for this purpose, e.g., an enzyme. Synthetic methods for coupling antibodies or antibody fragments to one another, or to enzyme labels, or to carriers, are well known in the art and need not be litanized here. See, for example, Ishikawa et al., J. of Immunoassay 4(3):209-327 (1983).

A particularly advantageous method for preparing an enzyme-labeled conjugate has been developed in the course of work on the present invention. This preferred approach yields a prepolymerized enzyme trimer, and is especially advantageous where the enzyme is alkaline phosphatase. The trimer is produced by reaction of the enzyme in the presence of a carbohydrate cross-linking agent, e.g., a dihydrazide such as adipic dihydrazide. By controlling the amounts of reactants and the reaction time and conditions, a trimer is produced which is cleanly separably by gel filtration from unreacted components and dimer, tetramer, and other polymerized forms of the enzyme. This defined, substantially pure component can be further modified with a homo- or heterobifunctional reagent in order that an antibody or antibody fragment can be attached. Trimeric alkaline phosphatase, polymerized through the carbohydrate moiety of the enzyme, is, in particular, stable and can be prepared and stored in large quantities.

The preparation of the antibody component of the labeled antibody reagent can be readily accomplished by the ordinary worker in the field. As used herein the terms antibody and antibody reagent are intended to encompass any material, however obtained, which comprises an antibody combining site. Thus, included in the intended meaning of such terms are whole immunoglobulins as well as appropriate fragments or polyfunctionalized forms thereof. When in the form of whole immunoglobulin, it can belong to any of the known classes and subclasses such as IgG, IgM, and the like. Any fragment of any such immunoglobulin which retains a functional antibody combining site can also be employed, for instance, the fragments of IgG conventionally known as Fab, Fab', and F(ab')₂. In addition, aggregates, polymers, derivatives, conjugates, and hybrids of immunoglobulins or their fragments can also be used where appropriate.

The immunoglobulin source of the antibody reagent can be derived by any available technique. Most commonly, such techniques will be conventional polyclonal antiserum production or monoclonal antibody techniques (the recently developed combinatorial expression library approach can also be used - Science (Dec 8, 1989) pp. 1275-81). Where an immunogen is used to generate antibody to the desired Chlamydia antigen in a host animal, such immunogen can be whole, intact, treated (e.g., denatured, reduced, oxidized, etc.) or untreated, Chlamydia EBs or RBs; a mixture obtained by disrupting or lysing such cells or a purified fraction or substance therefrom, e.g., the major outer membrane protein or LPS; a synthetically prepared peptide, polypeptide, carbohydrate, glycopeptide, lipopeptide, or the like, comprising an epitope specific to Chlamydia; and so forth as is known in the art. Antiserum obtained in this manner can be used directly in the preparation of the labeled antibody conjugate or can be subjected to further treatments and purifications such as affinity purification with a suitable ligand. e.g., a synthetic peptide mimic or analog of the Chlamydia epitope of interest.

Monoclonal techniques can also be used. For example, mice can be immunized with an immunogen as described above. Lymphocytes removed from immunized mice, optionally pre-screened for production of antibodies against or specific for Chlamydia, are then fused under conventional conditions with murine myeloma cells, and the resulting hybridomas screened for secretion of antibodies of desired specificity, affinity, and so forth.

Any useful type of immunoassay label can be used in the present invention. Typically used are enzymes, fluorescers, dyes (such as phycobiliproteins), dye aggregates (such as metal sols, dye polymers, colored latex, and the like), chemiluminescers, and so forth. Enzymes are particularly useful in providing sensitive detection of the retained immune complexes and are exemplified by peroxidase, beta-galactosidase, and alkaline phosphatase, the latter generally being preferred. Particularly useful labeled conjugates comprise a core of multiple enzyme molecules coupled with multiple antibodies or antibody fragments.

In general, the relative molecular size and net charge (determined using standard gel electrophoresis methods) of the labeled antibody reagent has been found to be significant in obtaining separation of bound and unbound reagent by passage through the depth filter. While it should be clearly understood that criticalities in the selection of relative size of the labeled reagent will be dependent upon the choices of Chlamydia antigens to be detected, the characteristics of the separation filter, the effect of any pre-filtration step on the test mixture, and other factors, normally it has been found that the relative molecular size of the labeled antibody reagent should be operably between about 200 kilodaltons (kd) and about 3000 kd, while it will generally be preferable to be between about 400 kd and about 1500 kd. Conjugates of approximately 1000 kd have been found to be particularly useful.

The time of incubation of the labeled antibody reagent and the exposed antigens can vary widely according to the relative avidity of the antibody reagent, the size and valency of the labeled conjugate, and the needs or desires of the user of the test. Typically, half of maximum binding rates can be achieved in under about 30 minutes, and accordingly adequate sensitivity can be obtained with incubation times in the range of 15-20 minutes. Because the present method involves a minimum of manipulative steps, the overall assay time can be less than about 25 minutes, and even less than about 15 minutes.

The separation filter is selected to be substantially impervious to the formed immune complexes between the Chlamydia antigens and the labeled antibody reagent while not retaining any significant amount of unbound labeled reagent. The latter will constitute undesirable background signal unrelated to the presence of Chlamydia in the test sample and can be held below acceptable limits by appropriate selection of reagents, assay protocol, and the separation filter. The mechanism of separation is generally not critical, although in principle the separation is seen as being the result of filtration, i.e., as a physical block to the passage of the immune complexes through the filter. Other forces can be involved as well, including ionic, hydrophobic, hydrogen bonding, or other adsorption phenomena. Similarly, the complexes can be retained on the surface and/or within the pores or internal structure of the filter. The critical net result is that the filter is impervious to the complexes.

In practice, glass fiber depth filters have been found to be most suitable. Conventional membrane filters have generally produced unacceptably slow flow rates. The useful glass fiber filters include those composed of borosilicate or quartz, fired or unfired. Particular examples are the 1-ply depth filters from the DBS Whatman series of glass fiber filters (Whatman Paper Ltd., Kent, UK). Also useful are the Whatman PD008 series of glass filters containing polyvinyl alcohol (PVA) as a strengthener, as well as, 2-ply Whatman filters, e.g., PD008-8B, GD1, and GD2. Glass and quartz filters available from Microfiltration Systems, Inc., Dublin, CA, USA, under the designations GC-50, GB100R, GD-120 and QR100 have also been found to be useful. While, as above, criticalities in the porosity of the separation filter will, in general, be dependent on the other parameters of the present assay, normally porosities in the range of from about 1 to about 5 microns have been found to be preferable, although a more preferred range of selection can be from about 1.5 to about 3 microns (with porosities around 1.6 to 2 microns particularly useful).

After passing the liquid test mixture through the separation filter, the filter is washed to remove any significant amounts of unbound labeled reagent. The composition of suitable wash solutions is well known in the art. Generally, the wash solution will comprise a buffer compatible with retaining immune complexes and particularly compatible with the label associated therewith. The pH of the buffer will be selected with these considerations in mind. In general, alkaline pH has been found to be desirable to increase retention of immune complexes while slightly acid pH reduces background. The buffer can contain other ingredients as well such as salt, detergents, stabilizers, and the like. Detergents in particular can be important in stabilizing enzyme labels, e.g., Triton X-100 (0.1%) has been found to reduce background significantly in the case of alkaline phosphatase labels. Also, a particularly useful wash buffer for use with bloody specimens is 0.5 M NaCl, 0.1 M TRIS (pH 6-7), and 0.1% Triton X-100.

After passing the liquid test mixture through the filter and washing the filter, the final critical step in the method is the detection of label retained with by the filter. This will be accomplished according to the type of label selected. In the preferred case of enzyme labels, the detection step will involve the addition of a suitable substrate to the filter, such as a chromogenic substrate (one which is acted upon by the enzyme to produce a distinctly colored product). Further, chromogenic substrates which produce product that becomes immobilized in or on the filter provide particularly concentrated colors which enhance the sensitivity and readability of the assay. Examples are the known alkaline phosphatase chromogenic substrates whose products are water insoluble and accordingly precipitate on and in the filter upon reaction with the enzyme label. These are parameters within the ordinary skill in the art.

The present invention also provides test kits and test devices for performing the half-sandwich method. Such will comprise (a) the sample processing agent, optionally contained within a sample processing tube or other vessel, (b) the labeled antibody reagent, and (c) the separation filter. Optional components of a test kit include a pre-filter device, a wash solution, and substrate solution (in the case of enzyme labels). Such means for performing the present method will comprise the sample processing agent and the labeled antibody in separate suitable containers. The separation filter will preferably be formed as part of a processing device or apparatus for performing a single or multiple assays and which facilitates handling, particularly the application and passage of liquids through the filter.

In a preferred embodiment, one that is particularly designed for performance of the test by relatively untrained individuals, the separation filter is housed in a small free-standing plastic device which exposes a suitable circular or other shaped area of the filter for application of the test sample, the wash solution, and the developing solution. Preferably, the device will also house an absorbent reservoir enclosed below but in liquid flow contact with the filter in order to facilitate flow and containment of such liquids after they have performed their functions in passing through the separation filter. Such devices are currently well known (see, for example, U.S. Patent Nos. 4,623,461; 4,632,901; 4,693,834; 4,727,019; and 4,818,677).

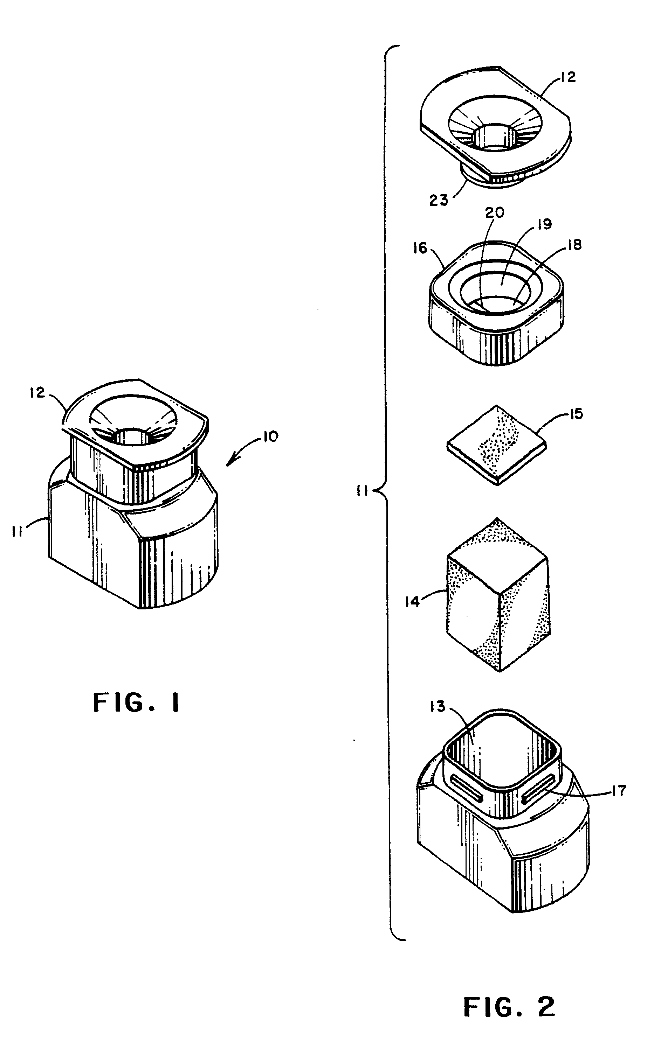

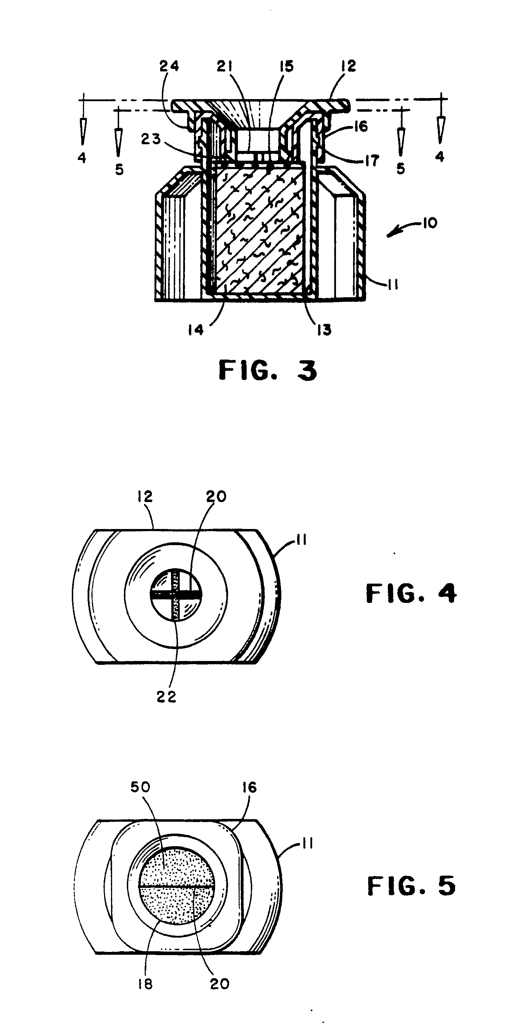

A particularly unique device that can be applied to the present Chlamydia assay is illustrated in the drawings. As illustrated in Figs. 1-3, test device 10 comprises a base member 11 and a removable funneling element 12. Base member 11 has a cavity 13 which accommodates both absorbent block 14 (formed of a highly water absorbent material such as Transorb· reservoirs available from American Filtrona Co., Richmond, VA, USA), and glass fiber filter 15 (e.g., cut from PD-008 12B 100 available from Whatman Paper Ltd., Kent, UK). Cap 16 is molded with an inner groove to snap fit onto ridges 17 on base member 11 and has a circular aperture 18 defined by the bottom edge of wall 19. The combined height of the absorbent block 14 and the glass fiber filter 15 is sufficient that they are held firmly in place by the bottom edge of wall 19 in the snap fit cap 16. Attached to the bottom edge of wall 19 in cap 16 and extending diametrically across aperture 18 is thread element 20 which is incorporated with a control reagent. As a result, when cap 16 is snap fit onto base member 11, thread control element 20 is held in physical contact with the surface of glass fiber filter 15 exposed in cap aperture 18. Fig. 5 is a top plan view of device 10 with funneling element 12 removed. Thread control element 20 is shown extending across surface 50 of glass fiber filter 15 exposed by cap aperture 18.

With reference to Figs. 3 and 4, bottom plate 21 of funneling element 12 has an aperture 22 which is shaped as a cross or plus sign (" + "). Further, funneling element 12 has an annular flange 23 and pegs 24 to hold same firmly in place when inserted into cap 16. Pegs 24 also serve to orient the funneling element when fit into cap 16 so that one of the cross bars of cross-shaped aperture 22 is coincident with control thread 20 extending across the surface of glass fiber filter 15 exposed in cap aperture 18.

An illustrated preferred use of test device 10 in the present assay method for the detection of Chlamydia is as follows. Separate from device 10, a test specimen such as a cervical or urethral swab is immersed in a specimen processing liquid which releases and exposes Chlamydia antigens, if any, present in the specimen. The swab is removed, leaving a liquid mixture which, in the case of a specimen that is positive for Chlamydia, contains multivalent Chlamydia antigens. Enzyme-labeled antibody reagent (as described herein) is added to the liquid mixture and incubated for a suitable period, e.g., 20 minutes, to allow formation of immune complexes with the Chlamydia antigens. The liquid test mixture is then poured, optionally after being pre-filtered as described above, into funneling element 12 in assembled test device 10 thereby flowing through cross-shaped aperture 22, in contact with control thread 20, through glass fiber filter 15, and into absorbent block 14.

In accordance with the present invention, labeled immune complexes are consequently retained by glass fiber filter 15 in a cross-shaped pattern on the exposed surface of such filter 15. Next, a wash solution is poured into the test device to wash the exposed surface of filter 15 and carry unbound labeled antibody and potentially interfering substances into absorbent block 14. Finally, an appropriate substrate/chromogen indicator solution is poured into the device resulting in the formation of color on the thread control element (if the test procedure has been followed correctly and the reagents are functional) and, in the case of a positive specimen, on the exposed filter surface in the pattern of a cross or plus sign.

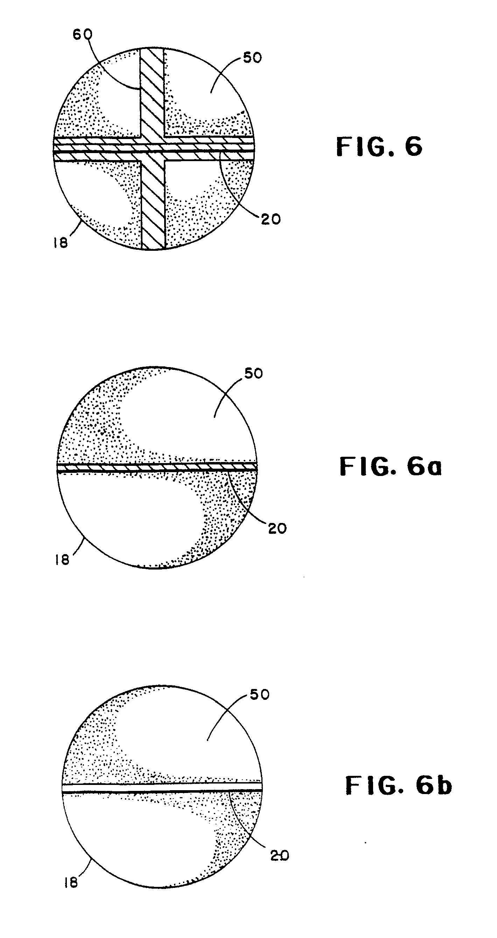

Figs. 6, 6a, and 6b illustrated possible test results appearing on the exposed surface 50 appearing within cap aperture 18. Fig. 6 illustrates the condition of the exposed test surface upon the conclusion of an assay on a specimen positive for Chlamydia. The cross-shaped area 60 exposed on the test surface by the funneling element, as well as control thread 20, has developed color (indicated by cross-hatching). Fig. 6a illustrates the condition of the exposed test surface upon the conclusion of an assay on a truly false specimen. No colored response appears on the exposed surface of the test surface, however, control thread 20 has turned color to indicate that the test procedure was correctly followed and the reagents were functional. Finally, Fig. 6b illustrates the condition of the exposed test surface upon the conclusion of an assay that was run incorrectly or with a nonfunctioning reagent. No colored pattern appears either on the exposed surface of the filter or on the control thread element indicating a false negative result. Such a test result calls for a repeat of the assay.

The present invention will now be illustrated, but is not intended to be limited by, the following examples.

7

EXAMPLE

A. Preparation of Labeled Antibody Reagent

(1) Preparation of 22 Amino Acid Peptide-Sepharose 4B Affinity Support

The affinity support used for purification of goat polyclonal antibodies which bind the serovar-specific epitope (Stephens, et al., "A Species-Specific Major Outer Membrane Protein Domain," in Chlamydia Infections, Oriel et al. eds., Cambridge University Press, Great Britain 1986, pp. 110-113) of chlamydia was prepared by the following procedure. Four grams of AH-Sepharose® (Pharmacia, Piscataway, NJ, USA) was swollen in 50 mL of 0.5 M sodium chloride, washed with 200 mL of same; and then washed with 200 mL of 0.1 M sodium phosphate, pH 7.0 containing 0.15 M sodium chloride and 1.0 mM EDTA (PBS·EDTA). The resin was activated with 120 µmoles of sulfosuccinimidyl 4-(N-maleimidomethyl)cyclohexane-1-carboxylate (Sulfo-SMCC; Pierce, Rockford, IL, USA) in 25 mL of PBS·EDTA. After shaking for two hours at ambient temperature, the maleimide-activated resin was washed with 300 mL of PBS·EDTA and resuspended in 16 mL of the same. The resin was then reacted with 19.8 mg of a 22 amino acid peptide containing the specific-specific epitope described by Stephens et al., (1986) with 6 spacer residues terminating at the carboxyl end with cysteine (OCS Laboratories, Inc., Denton, TX, USA). The reaction proceeded overnight with continuous mixing at ambient temperature. The peptide-Sepharose was washed with 300 mL PBS-EDTA with 0.1% Tween-20 (Sigma Chemical Co., St. Louis, MO, USA) to remove unreacted peptide. The resin was then resuspended in 30 mL of 6 M guanidine·HCl, pH 5.5, and stored at 4°C.

(2) Purification of Goat Antibody to Chlamydia

Antiserum was obtained from goats immunized with UV-inactivated elementary bodies. After clarification, the IgG was isolated by precipitation with a 33% saturation of ammonium sulfate for 2 hours at ambient temperature. The precipitate was collected and redissolved in 0.1 M sodium phosphate, pH 7.0 containing 0.15 M sodium chloride (PBS) buffer. The IgG was then dialyzed against 50 volumes of PBS; the dialysis buffer was changed twice. After the IgG was clarified by filtration through a 0.8 micron Acrodisc filter (Gelman Sciences, Inc., Ann Arbor, MI, USA), 475 mg in 18 mL PBS was loaded onto a 6 mL peptide-Sepharose column (described in part 1 above) which had been equilibrated with PBS at ambient temperature. The absorbance at 180 nm was monitored during this and subsequent steps. After washing with PBS, the peptide-specific IgG was eluted sequentially with 0.1 M sodium acetate, pH 4.0; 0.1 M glycine, pH 3.0; and 6 M guanidine·HCl, pH 7.0. The fractions were neutralized with 1 M Bicine, pH 8.3 and the yields are summarized in Table 1.

## TABLE 1

| Fraction | mg IgG | % Yield |
|----------|--------|---------|
| pH 4 | 9.6 | 2.3 |
| pH 3 | 10.2 | 2.4 |
| guanidine·HCl | 2.3 | 0.5 |

(3) Preparation of the Antibody-Alkaline Phosphatase Conjugate

Beef mucosa alkaline phosphatase (Scripps Laboratories, San Diego, CA, USA) was thiolated as follows: 5.5 mg was reacted with 1.96 µmoles of 2-iminothiolane (Pierce, Rockford, IL, USA) in 1.0 mL of buffer containing 0.4 M triethanolamine, pH 8.0 and 0.1 mM EDTA. After one hour at ambient temperature, the reaction mixture was applied to a fresh 28 mL Bio-Gel P-6DG (Bio-Rad; Richmond, CA, USA) column equilibrated with PBS. The thiolated enzyme eluted at the void volume (5.5 mL). The number of thiols was determined to be 17.4 moles per mole enzyme. The thiols were then allowed to oxidize at ambient temperature (4.3 hours) until only 1.4 SH group per enzyme remained. At this time, the thiolated and polymerized alkaline phosphatase was conjugated with the affinity-purified IgG which had been modified with maleimides during the enzyme polymerization step.

Briefly, 5.04 mg of IgG (with 3% fluoresceinated IgG) was reacted with 378 nmoles of Sulfo-SMCC (above) in 0.93 mL of PBS. After 1.0 hours at RT, the reactants were separated on a similar Bio-Gel P6DG column. The maleimidated IgG eluted at the void volume, and was mixed with the polymerized enzyme as noted above.

The polymerized enzyme and maleimidated IgG were reacted overnight at $5^\circ$ C with stirring. After capping unreacted thiols with 2 mM N-ethylmaleimide (Pierce); the conjugate reaction was applied to a 415 mL Bio-Gel A-1.5 (2.5 x 84 cm) column equilibrated with a 0.1 M HEPES buffer (pH 6.9) containing 0.1 M sodium chloride, 0.1 M magnesium chloride, 0.2 mM zinc chloride and 0.05% sodium azide. The conjugate was eluted with the same buffer at 30 mL per hour. Fractions were assayed for fluorescein fluorescence at 512 nm (excitation at 492 nm) and alkaline phosphatase activity. Peak fractions were pooled and supplemented with 5 mg/mL of both Triton X-100 (Mallinckrodt, Paris, KY, USA) and protease-free bovine serum albumin (Miles Inc., Kankakee, IL, USA). The conjugate was filtered through 0.2 micron Acrodisc and 0.5 μg/mL leupeptin (Boehringer Mannhein, Indianapolis, IN, USA) was added. The antibody to enzyme ratio was determined to be 1.7 to 1.0.

(4) A Preferred Synthesis for the Antibody-Alkaline Phosphatase Conjugate

This method for synthesizing the antibody-enzyme conjugate avoids side reactions that can occur during the polymerization step, consequently, the synthesis is more reproducible. In addition, the conjugate is also less sensitive to substances which may be found in either the specimen or included in the extraction buffer to facilitate specimen processing and expose the Chlamydial organism and its epitope.

Prior to polymerizing the enzyme, it must be extensively dialyzed to remove all traces of glycerol. To that end, 17 mg of alkaline phosphatase in 1.0 mL was dialyzed against 250 mL of 0.1 M sodium acetate, pH 5.5 containing 0.1 M sodium chloride at $5^\circ$ C. The buffer was changed thrice. The dialyzed enzyme was then oxidized with 6.7 mg of sodium meta-periodate for 30 minutes in ice and in the dark. The reaction was then stopped by reaction with 0.1 mL of glycerol for 15 minutes. After dialyzing the peroxidized enzyme as before, the enzyme was polymerized with 2.8 moles of adipic dihydrazide (Aldrich, Milwaukee, WI, USA) per mole of alkaline phosphatase. After reacting overnight at $4^\circ$ C, the reaction mixture was applied to a 1.5 x 110 cm Bio-Gel A-0.5 (Bio-Rad) column previously equilibrated with 0.1 M sodium phosphate, pH 7.0 and 0.1 M sodium chloride. The column was run at $5^\circ$ C at 30 mL per hour. Two (2) mL fractions were collected. The active polymerized fractions peaked at #34. Unpolymerized enzyme peaked to a lesser degree in fraction #50. The polymerized enzyme was pooled, concentrated and stored refrigerated. The relative MW is approximately 420,000 and is therefore a trimer of the native enzyme.

The trimerized alkaline phosphatase (2.7 mg) was reacted with a 50-fold molar excess of N-sulfosuccinimidyl(4-iodoacteyl)aminobenzoate (Sulfo-SIAB; Pierce) introduced in 97 μL of dimethylformamide to 0.40 mL of enzyme in 0.5 M triethanolamine, pH 8.0. The reaction proceeded for 1.25 hour at ambient temperature. The modified enzyme was separated from unreacted Sulfo-SIAB on a small 1.0 x 22 cm Bio-Gel P6DG column equilibrated with 0.05 M potassium phosphate, pH 7.3 and 0.15 M sodium chloride. After concentration to less than 1 mL on a Centricon™ 30 microconcentrator (Amicon). The enzyme was kept on ice until use.

Affinity-purified goat antibody (4 mg) was mixed with 3% fluoresceinated IgG and thiolated at 50 to 100-fold molar excess of 2-iminothiolane in a 1.0 mL reaction also containing 0.1 M phosphate, 0.15 M NaCl, pH 7.3; 0.20 M triethanolamine, pH 8.0; 2 mM EDTA, and dithiothrietol added in the same molar excess with the 2-iminothiolane. After 1 hour at ambient temperature, the thiolated antibody was desalted on a fresh 1.5 x 22 cm Bio-Gel P6DG column equilibrated with a buffer containing 0.05 M potassium phosphate, pH 7.3, 0.15 M sodium chloride and 1 mM EDTA. After elution with the same buffer, the thiolated antibody was concentrated to 1.0 mL in a Centricon concentrator.

Iodoacetylated alkaline phosphatase trimer (2.7 mg in 1.0 mL) was reacted with 3.0 mg (1.0 mL) of thiolated antibody. The reaction also contained 100 μL of 0.5 M triethanolamine, pH 8; and 100 μL of a mixture containing 0.3 M sodium chloride, 20 mM magnesium chloride, and 0.4 mM zinc chloride. The final concentration of EDTA was 0.5 mM. The reaction proceeded with stirring at ambient temperature for 30 minutes followed by overnight reaction at $5^\circ$ C with the same buffer described in part 3 above. The flow rate was 31 mL per hour. Twenty drops were collected per fraction. Fractions were assayed for fluorescence and enzyme activity, and combined into two pools. Triton X-100 and bovine serum albumin were added to each pool at 5 mg per mL. The conjugates were then filtered through a 0.22μ Acrodisc and characterized. The ratio of IgG to alkaline phosphatase is 1.8.

B. Performance of Chlamydia Immunoassay

A standardized preparation of Percoll-purified Chlamydia elementary bodies (EB's) was serially diluted in 100 mM Tris-HCl buffer (pH 8.0) containing 100 mM NaCl and 5 mg/mL bovine serum albumin (BSA). Twenty (20) microliters of each dilution were added to 400 $\mu$L of specimen processing buffer, consisting of 100 mM Tris-HCl buffer (pH 9.0) containing 0.5% Triton X-100, 0.5% n-lauryl sarcosine, 0.5 M NaCl and 5 mg/mL BSA. Immediately, 50 $\mu$L of the polyclonal antibody/alkaline phosphatase conjugate as prepared in part A above (in 100 mM HEPES buffer (pH 6.9) containing 100 mM NaCl, 200 $\mu$M ZnCl$_2$, 10 mM MgCl$_2$, and 5 mg/mL BSA) were added. The mixture was incubated 20 minutes at room temperature. During this period assay devices were set up as described in the text above. They contained a Whatman PD-008 12B 100 glass fiber filter, an S&S #24 filter as a cushion, and a Transorb (American Filtrona) liquid reservoir. At the end of the incubation period, the reaction mixture was poured through the funneling element. The funneling element was removed and the reaction filter was washed with 1 mL 100 mM Tris-HCl (pH 8) containing 0.5 M NaCl and 0.1% Triton X-100. Finally, 50 $\mu$L substrate (5 mM 5-bromo-4-chloroindoylphosphate (BCIP) dissolved in 150 mM bis-Tris propane (pH 9.8) containing 75 mM MgCl$_2$ was applied to the reaction filter. After 5 minutes, the presence or absence of a blue cross on the reaction filter was determined visually.

For comparison purposes, 20 $\mu$L aliquots of each dilution were also applied to Syva MicroTrack slides (Syva Company, Palo Alto, CA, USA). The slides were dried and the remainder of the procedure followed the standard Syva protocol. In addition, another set of 20 $\mu$L aliquots were run in Abbott TestPack Chlamydia assay kits (Abbott Laboratories, North Chicago, IL, USA). The manufacturers' instructions were followed except that the pre-filtration step was omitted. Protein concentration was measured using the Pierce BCA protein assay kit (Pierce Chemical Co., Rockford, IL, USA). The results are shown in Table 2.

## Table 2

| EB protein (ng/assay) | 0 | 1.25 | 2.5 | 5 | 10 | 20 |
|---|---|---|---|---|---|---|
| Syva MicroTrack (EB/field)[1] | 0 | 1.6 | 1.4 | 4.4 | 7.2 | ND[2] |
| Abbott TestPack Chlamydia | - | ND | - | + | + | + |
| Half-sandwich Assay | - | - | + | + | + | + |

[1] Syva results are the average number of EB's counted over 10 fields

[2] ND: Not Determined
+: Positive test result
-: Negative test result
+: Intermediate, faint positive test result

The present invention has been particularly described and exemplified above. Clearly, many other variations and modifications of the invention can be made without departing from the spirit and scope hereof.

## Claims

1. A method for detecting the presence of <u>Chlamydia trachomatis</u> in a test sample in which the test sample is treated to form a liquid test mixture containing exposed multivalent C. trachomatis antigens and said antigens are detected,

(a) characterized in that the exposed multivalent C. trachomatis antigens are contacted with an antibody reagent that binds to said C. trachomatis antigens to form immune complexes, said antibody reagent comprising a detectable label,

(b) the resulting liquid test mixture is passed through a glass fiber depth filter that is substantially impervious to said immune complexes but which does not retain a significant amount of labeled antibody reagent that is not bound in such immune complexes,

(c) a wash solution is passed through the glass fiber filter effective to remove substantially all unbound labeled antibody reagent present thereon after completion of step(b), and

(d) label retained by the filter is detected.

2. The method of claim 1 wherein the test sample is a clinical specimen, such as a urogenital specimen, comprising human cells, and wherein such clinical specimen is treated to rupture human cells in the sample sufficiently to release C. trachomatis therefrom and to disrupt the cytoplasimic membrane of any such released C. trachomatis to expose multivalent antigens.

3. The method of claim 2 wherein the C. trachomatis antigens exposed comprise (a) an outer membrane protein antigen, and wherein the clinical specimen is treated with a specimen processing liquid having a pH buffered to between about 8 and about 9 and optionally comprising a reducing agent, an oxidizing agent, and/or a detergent, or (b) a lipopolysaccharide antigen, and wherein the clinical specimen is treated with a specimen processing liquid having a pH buffered to between about 6.5 and about 9 and optionally comprising a reducing agent, an oxidizing agent, and/or a detergent.

4. The method of any of claims 1 to 3 wherein prior to step (b), and preferably after the test sample has been treated to expose said C. trachomatis antigens but before contact with said antibody reagent, the liquid test sample or mixture is passed through a filter to remove extraneous sample debris, thereby pre-filtering the test medium.

5. The method of any of claims 1 to 4 wherein the glass fiber depth filter has a porosity of between about 1 and about 5 microns, and preferably between about 1.5 and about 3.0 microns.

6. The method of any of claims 1 to 5 wherein step (b) is accomplished by adding the test mixture resulting from step (a) to a test device comprising said glass fiber depth filter, one side of which is exposed for receiving the mixture and the other is in liquid flow contact with an absorbent reservoir, whereby said mixture flows through the filter and is absorbed into said absorbent material.

7. The method of any of claims 1 to 6 wherein the label is an enzyme.

8. The method of claim 7 wherein the labeled antibody reagent comprises one or more enzymes chemically coupled to one or more IgG antibodies or fragments thereof, and has a total relative molecular weight of between about 200 and about 3000 kilodaltons, preferably between about 400 and about 1500 kilodaltons.

9. The method of claim 7 or 8 wherein the presence of the enzyme label on the filter is detected by passing a solution containing a chromogenic substrate for the enzyme through the filter and observing color formed on the filter.

10. A test kit for detecting the presence of Chlamydia trachomatis in a test sample, the test kit comprising:

(1) a container holding a liquid sample processing agent capable of exposing multivalent C. trachomatis antigens,

(2) a container holding an antibody reagent capable of binding to said C. trachomatis antigens to form immune complexes, said antibody reagent comprising a detectable label, and

(3) a glass fiber depth filter that, upon passage of a liquid test mixture resulting from the combination of said labeled antibody reagent and test sample treated with said sample processing agent, is substantially impervious to said immune complexes but which does not retain a significant amount of labeled antibody that is not bound in such immune complexes.

FIG. 1

FIG. 2

FIG. 3

FIG. 4

FIG. 5

FIG. 6

FIG. 6a

FIG. 6b

14